# EUROPEAN PATENT APPLICATION

(11) **EP 4 431 048 A1**
(43) Date of publication of application: **18.09.2024**
(21) Application number: 21951109.4
(22) Date of filing: 07.12.2021
(51) Int. Cl.: A61B 34/37

(54) **MAIN MANIPULATOR AND SURGICAL ROBOT CONTROL DEVICE**

(30) Priority: 08.11.2021 CN 202111311759
(71) Applicant: Harbin Intelligent Surgery Equipment Co., Ltd., Harbin, Heilongjiang 150000 (CN)
(72) Inventor: LI, Yicheng, Harbin, Heilongjiang 150000 (CN); LIU, Chang, Harbin, Heilongjiang 150000 (CN); LIANG, Yunlei, Harbin, Heilongjiang 150000 (CN); WANG, Jianguo, Harbin, Heilongjiang 150000 (CN); DING, Hui, Harbin, Heilongjiang 150000 (CN); ZHANG, Haizhu, Harbin, Heilongjiang 150000 (CN)
(74) Representative: Murgitroyd & Company
(86) International application number: PCT/CN2021/135985
(87) International publication number: WO 2023/077605

(57) **Abstract**

The invention provides a main manipulator and surgical robot control equipment, and relates to the technical field of medical equipment. The main manipulator comprises a root structure, an arm structure and a hand structure. The root structure is suitable for being connected to a chassis of a console. One end of the arm structure is connected to the root structure, and the other end of the arm structure is connected to the hand structure. After the root structure is connected to the chassis, the hand structure is suitable for being exposed from the table top of the console. According to the the invention, an operator can conveniently operate the hand structure in either a standing position or a sitting position. At the same time, the root structure and the arm structure can be hidden below the table top of the console, which not only does not hinder the operator from observing a display, but also can make the position of the display be reasonably set according to the need so as to meet the optimal observation distance, thereby making the operation feeling of the main manipulator of the surgical robot control equipment better.

## Description

### Technical Field

The invention relates to the technical field of medical equipment, and in particular to a main manipulator and surgical robot control equipment.

### Background Art

In recent years, as minimally invasive surgery has been more and more widely used in clinical surgery, the application of minimally invasive surgical robot systems is also increasing. A main manipulator is an important part of the minimally invasive surgical robot system and a carrier of information transmission between a surgeon and a slave robotic arm. In the process of minimally invasive surgery, the surgeon usually operates the main manipulator on control equipment such as a doctor's console, remote equipment or a robot control platform to drive a slave robotic arm of the surgical robot to perform surgical procedures.

In the existing control equipment, an annular arm is usually arranged above the console, and the main manipulator is arranged on the annular arm in a hoisting manner. Whether a display is designed at the front or at the rear, the control equipment installed in this way is difficult to provide a comfortable operation posture for operators, or the main manipulator easily interferes with the operator's observation of the display during operation, which makes the operators feel bad in operation.

### Summary of the Invention

The invention solves the problem of how to make the operation feeling of a main manipulator of surgical robot control equipment better.

In order to solve the above problem, the invention provides a main manipulator, which comprises a root structure, an arm structure, and a hand structure. The root structure is suitable for being connected to a chassis of a console. One end of the arm structure is connected to the root structure, and the other end of the arm structure is connected to the hand structure. After the root structure is connected to the chassis, the hand structure is suitable for being exposed from the table top of the console.

Optionally, the root structure includes a position shoulder deflection joint which is suitable for being connected to the chassis and realizing a position shoulder deflection operation.

Optionally, the root structure further includes a position shoulder pitching joint which is suitable for being connected to the position shoulder deflection joint and realizing a position shoulder pitching operation.

Optionally, the arm structure includes a big arm, a small arm, and a position elbow pitching joint. One end of the big arm is connected to the position shoulder pitching joint, and the other end of the big arm is connected to the small arm through the position elbow pitching joint. The position elbow pitching joint is suitable for realizing a position elbow pitching operation.

Optionally, the hand structure includes a posture redundancy deflection joint, a posture pitching joint, a posture deflection joint, and a posture rotation joint which are connected in sequence through a skeleton structure. The posture redundancy deflection joint is connected to one end, far away from the position elbow pitching joint, of the small arm, and is suitable for realizing a posture redundancy deflection operation. The posture pitching joint is suitable for realizing a posture pitching operation. The posture deflection joint is suitable for realizing a posture deflection operation. The posture rotation joint is suitable for realizing a posture rotation operation.

Optionally, the joint axes of the posture redundancy deflection joint, the posture pitching joint, the posture deflection joint, and the posture rotation joint intersect at one point.

Optionally, the hand structure further includes a clip pinching joint which is connected to the posture rotation joint and is suitable for realizing a clip pinching operation.

Compared with the prior art, the the invention has the following effects:
the root structure is connected to the chassis of the console, one end of the arm structure is connected to the root structure, the other end of the arm structure is connected to the hand structure, and the hand structure is suitable for being exposed from the table top of the console, so that an operator can conveniently operate the hand structure in either a standing position or a sitting position. At the same time, the root structure and the arm structure can be hidden below the table top of the console, which not only does not hinder the operator from observing a display, but also can make the position of the display be reasonably set according to the need so as to meet the optimal observation distance, thereby making the operation feeling of the main manipulator of the surgical robot control equipment better.

Another objective of the invention is to provide surgical robot control equipment to solve the problem of how to make the operation feeling of the main manipulator of the surgical robot control equipment better.

In order to achieve the above problem, the technical solution of the invention is implemented as follows:
the surgical robot control equipment includes the console and the main manipulator as mentioned above; and after the root structure of the main manipulator is connected to the chassis of the console, the hand structure of the main manipulator is suitable for being exposed from the table top of the console.

Optionally, the surgical robot control equipment further includes a display module which is arranged on the console and located on one side of the main manipulator.

Optionally, the surgical robot control equipment further includes an armrest module which is arranged on the console and located on one side, away from the display module, of the main manipulator.

The surgical robot control equipment has the same advantages as the above-mentioned main manipulator with respect to the prior art, and the descriptions thereof are omitted herein.

### Brief Description of the Drawings

Fig.1 is a schematic structural diagram of a main manipulator according to an embodiment of the invention.
Fig. 2 is a schematic structural diagram of a main manipulator from another perspective according to an embodiment of the invention.
Fig. 3 is a schematic diagram of the hand structure movement of a main manipulator according to an embodiment of the invention.
Fig. 4 is a schematic structural diagram of surgical robot control equipment according to an embodiment of the invention.
Fig. 5 is a schematic structural diagram of surgical robot control equipment from another perspective according to an embodiment of the invention.
Fig. 6 is a schematic structural diagram of surgical robot control equipment from yet another perspective according to an embodiment of the invention.

### Description of Reference Numerals:

1-root structure; 11-position shoulder deflection joint; 12-position shoulder pitching joint; 2-arm structure; 21-big arm; 22-small arm; 23-position elbow pitching joint; 3-hand structure; 31-posture redundancy deflection joint; 32-posture pitching joint; 33-posture deflection joint; 34-posture rotation joint; 35-clip pinching joint; 100-console; 101-chassis; 102-stand column; 200-display module; 201-main display; 202-auxiliary display; 300-armrest module; 301-armrest; 302-key; 303-touch screen; 400-pedal; 500-push hand; and 600-voice device

### Detailed Description of the Invention

To make the above objects, features and advantages of the invention clearer and more comprehensible, specific embodiments of the the invention will be described in detail below with reference to the accompanying drawings.

In the description of the invention, it should be understood that the forward direction of "X" in the accompanying drawings represents the left side, and correspondingly the reverse direction of "X" represents the right side; the forward direction of "Y" represents the front, and accordingly the reverse direction of "Y" represents the rear; the forward direction of "Z" represents the above, and correspondingly the reverse direction of "Z" represents the below.; the orientation or position relationships indicated by terms "Z", "Y", "Z" and the like are based on orientation or position relationships shown in the accompanying drawings of the description, are only for the convenience of describing the invention and simplifying the description, and do not indicate or imply that the specified device or component must have a specific orientation and must be constructed and operated in the specific orientation, so that it cannot be understood as a limitation to the invention.

The terms "first", "second" and "third" are only intended for description, but cannot be construed as indicating or implying relative importance or implicitly indicating the number of the specified technical features. In addition, the described specific features, structures, materials, or characteristics may be combined in a proper manner in any one or more of the embodiments or examples.

In order to solve the above problem, the embodiment of the invention provides a main manipulator, which includes a root structure 1, an arm structure 2, and a hand structure 3. The root structure 1 is suitable for being connected to a chassis 101 of a console 100. One end of the arm structure 2 is connected to the root structure 1, and the other end of the arm structure 2 is connected to the hand structure 3. After the root structure 1 is connected to the chassis 101, the hand structure 3 is suitable for being exposed from the table top of the console 100.

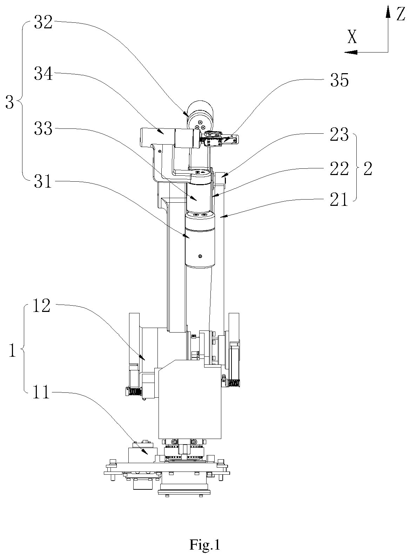

As shown in Fig. 1, Fig. 2, and Fig. 3, the root structure 1 adopts a structure with joints that can be bent or rotated, and can be connected to the chassis 101 of the console 100 by means of welding, riveting or bolted connection; the arm structure 2 can adopt a rigid structure or a structure with joints that can be bent or rotated; the hand structure 3 is connected to the arm structure 2; and part or the whole of the hand structure 3 can be exposed from the table top of the console 100.

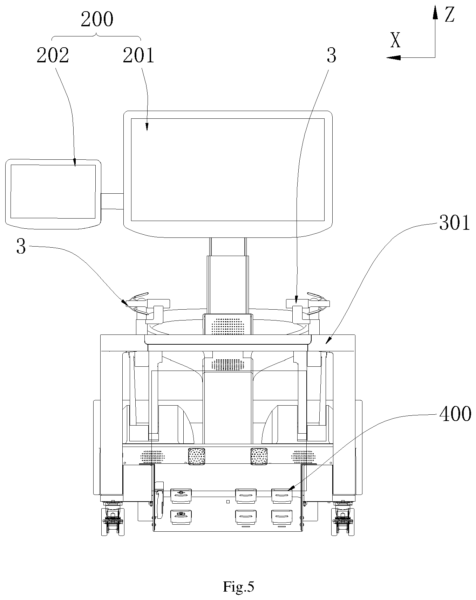

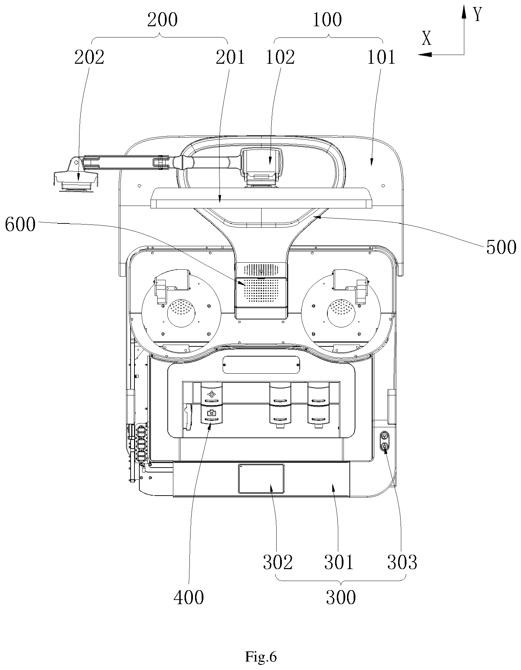

In the present embodiment, as shown in Fig. 5 and Fig. 6, the chassis 101 is provided with a mounting position, and the root structure 1 can be mounted at the mounting position through a mounting plate. After the root structure 1 is connected to the chassis 101, a movable part of the root structure 1 can be rotated or bent relative to the chassis 101. The arm structure 2 can also adopt a structure with joints that can be bent or rotated. After the hand structure 3 is connected to the arm structure 2, most of the hand structure 3 can be exposed from the table top of the console 100.

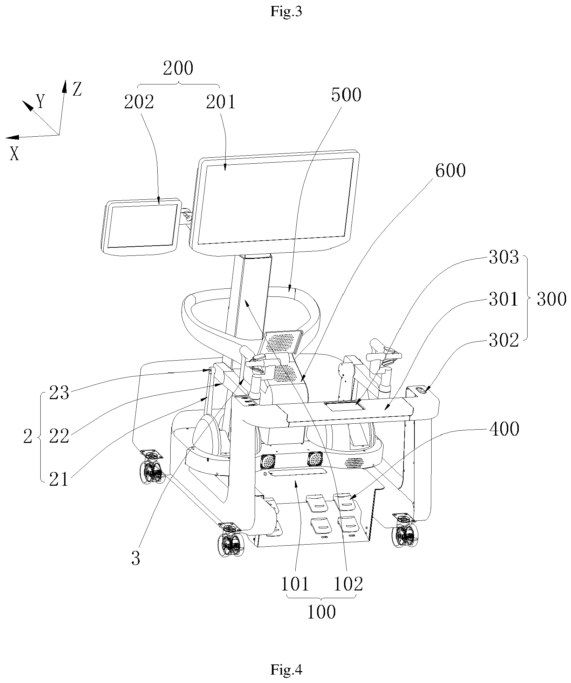

It should be noted that the table top of the console 100 can be of a solid structure, such as a platform, or a virtual plane. For example, when the console 100 is of an open structure, a certain horizontal operation surface located above the chassis 101 can be regarded as the table top of the console 100. Specifically, in the present embodiment, as shown in Fig. 4 and Fig. 5, the plane where the upper end surface of an armrest 301 is located can be regarded as the table top of the console 100.

In this way, the root structure 1 is connected to the chassis 101 of the console 100, one end of the arm structure 2 is connected to the root structure 1, and the other end of the arm structure 2 is connected to the hand structure 3, and the hand structure 3 is suitable for being exposed from the table top of the console 100, so that an operator can conveniently operate the hand structure 3 in either a standing position or a sitting position. At the same time, the root structure 1 and the arm structure 2 can be hidden below the table surface of the console 100, which not only does not hinder the operator from observing a display, but also can make the position of the display be reasonably set according to the need so as to meet the optimal observation distance, thereby making the operation feeling of the main manipulator of the surgical robot control equipment better.

Optionally, the root structure 1 includes a position shoulder deflection joint 11 and a position shoulder pitching joint 12. The position shoulder deflection joint 11 is suitable for being connected to the chassis 101 and realizing a position shoulder deflection operation. The position shoulder pitching joint 12 is suitable for being connected to the position shoulder deflection joint 11 and realizing a position shoulder pitching operation. The arm structure 2 includes a big arm 21, a small arm 22 and a position elbow pitching joint 23. One end of the big arm 21 is connected to the position shoulder pitching joint 12, and the other end of the big arm 21 is connected to the small arm 22 through the position elbow pitching joint 23. The position elbow pitching joint 23 is suitable for realizing a position elbow pitching operation. The hand structure 3 includes a posture redundancy deflection joint 31, a posture pitching joint 32, a posture deflection joint 33, a posture rotation joint 34, and a clip pinching joint 35 which are connected in sequence through a skeleton structure. The posture redundancy deflection joint 31 is connected to one end, far away from the position elbow pitching joint 23, of the small arm 22, and is suitable for realizing a posture redundancy deflection operation. The posture pitching joint 32 is suitable for realizing a posture pitching operation. The posture deflection joint 33 is suitable for realizing a posture deflection operation. The posture rotation joint 34 is suitable for realizing a posture rotation operation. The clip pinching joint 35 is connected to the posture rotation joint 34 and is suitable for realizing a clip pinching operation. The joint axes of the posture redundancy deflection joint 31, the posture pitching joint 32, the posture deflection joint 33, and the posture rotation joint 34 intersect at one point.

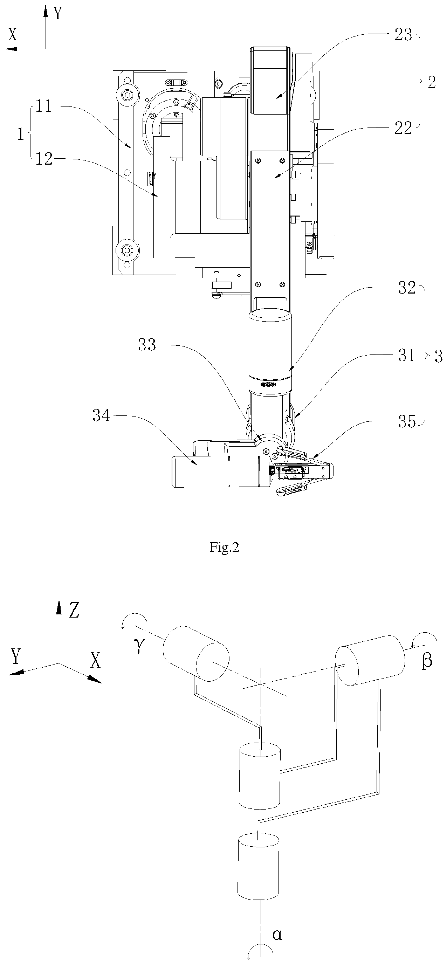

As shown in Fig. 1 and Fig. 2, in the present embodiment, the position shoulder pitching joint 12 is arranged above the position shoulder deflection joint 11, and is connected to a movable part of the position shoulder deflection joint 11. The position shoulder deflection joint 11 can drive the main body of the main manipulator to realize root rotation.

The position elbow pitching joint 23 is arranged above the position shoulder pitching joint 12, and is connected to the position shoulder pitching joint 12 by the big arm 21 composed of a quadrilateral link mechanism. The posture redundancy deflection joint 31 is arranged at one side of the position elbow pitching joint 23, and is connected to the position elbow pitching joint 23 by the small arm 22 composed of a rigid rod structure. The shoulder pitching joint 12 can drive the big arm 21 and the structure connected to the big arm 21 to pitch at a certain angle in the height direction. The elbow pitch joint 23 can drive the small arm 22 and the structure connected to the small arm 22 to pitch at a certain angle in the horizontal plane. The posture redundancy deflection joint 31 can drive the hand structure 3 to achieve posture redundancy deflection.

As shown in Fig. 1 and Fig. 4, the height direction refers to the Z-axis direction, and the horizontal plane refers to the plane parallel to the XY plane.

The posture pitching joint 32 is arranged above the posture redundancy deflection joint 31 and connected to the posture redundancy deflection joint 31 through a first L-shaped support. The posture pitching joint 32 can drive the hand structure 3 to achieve posture pitching. The posture deflection joint 33 is arranged on one side of the posture pitching joint 32 and connected to the posture pitching joint 32 through a second L-shaped support. The posture deflection joint 33 can drive the hand structure 3 to achieve posture deflection. The posture rotation joint 34 is arranged on one side of the posture deflection joint 33, and is connected to the posture deflection joint 33 through a third L-shaped support. The posture rotation joint 34 can drive the hand structure 3 to realize posture rotation. The clip pinching joint 35 is connected to the end of the posture rotation joint 34, and can realize a clip pinching operation.

It should be noted that as shown in Fig. 3, the posture redundancy deflection of the hand structure 3 and the posture deflection of the hand structure 3 mean that the hand structure 3 rotates along the alpha axis, the posture pitching of the hand structure 3 means that the hand structure 3 rotates along the beta axis, and the posture rotation of the hand structure 3 means that the hand structure 3 rotates along the gamma axis.

It is characterized in that the alpha-axis, the beta-axis and the gamma-axis intersect at one point, and each joint of the hand structure 3 moves around the intersection point of the axes in the movement process, which is equivalent to forming a spherical joint. This design mode satisfies Pipper's criterion (that is, the three adjacent joint axes of the robot intersect at one point or the three axes are parallel), which can make the hand structure 3 move very flexibly, and further simplify the kinematics analysis.

As shown in Fig. 1 and Fig. 2, in the present embodiment, the position shoulder deflection joint 11 includes a mounting plate, a motor, a main shaft, a first deceleration mechanism, an auxiliary shaft, an electromagnetic brake and an encoder. The first deceleration mechanism includes a deceleration belt and a deceleration gear. The main shaft and the auxiliary shaft are respectively arranged on the mounting plate in a penetration manner. The motor provides power for the position shoulder deflection joint 11, and drives the deceleration gear sleeved on the main shaft to rotate through the deceleration belt. The main shaft rotates along with the deceleration gear, and drives a speed-increasing gear arranged on the main shaft on the lower side of the mounting plate to rotate. The speed-increasing gear drives the auxiliary shaft to rotate through another belt. The encoder and the electromagnetic brake are arranged on the auxiliary shaft, and are located on the upper side and the lower side of the mounting plate respectively. The electromagnetic brake can provide braking for the position shoulder deflection joint 11. The encoder is used for detecting the position information of the position shoulder deflection joint 11 to start or stop the motor.

In this way, the output torque of the motor of the position shoulder deflection joint 11 can be increased by arranging the first deceleration mechanism and the encoder in the position shoulder deflection joint 11, so that the position shoulder deflection joint is more labor-saving and light for the operator in operation, at the same time, the flexibility and position accuracy of the position shoulder deflection joint 11 can be improved, and the return stroke clearance can be reduced, and thus, the position shoulder pitching joint 12 has good end operation feeling.

As shown in Fig. 1 and Fig. 2, in the present embodiment, the position shoulder pitching joint 12 includes two rotary tables, namely, a left rotary table and a right rotary table, which can rotate in opposite directions. The left rotary table and the right rotary table are coaxially arranged and installed on a supporting part of the position shoulder pitching joint 12 through the main shaft. The supporting part is connected to the movable part of the position shoulder deflection joint 11. The left rotary table and the right rotary table are powered by a motor respectively. The motors can drive the left rotary table and the right rotary table to rotate through a second deceleration mechanism. One link of the quadrilateral link mechanism is fixedly connected to the left rotary table, and the other link of the quadrilateral link mechanism is eccentrically connected to the right rotary table through a rotating shaft, so that the quadrilateral link mechanism can be driven by the rotation of the left rotary table and the right rotary table, and thus, the pitching of the position shoulder pitching joint 12 is realized.

In this way, the output torque of the motor of the position shoulder pitching joint 12 can be increased by arranging the second deceleration mechanism in the position shoulder pitching joint 12, so that the position shoulder pitching joint 12 is more labor-saving and light for the operator in operation, at the same time, the flexibility of the position shoulder pitching joint 12 can be improved, the return stroke clearance is reduced, the gravity, inertia force and frictional force of the position shoulder pitching joint 12 and an upper structure thereof are compensated, and thus, the main manipulator is more flexible in operation without a jamming phenomenon.

As shown in Fig. 1 and Fig. 2, in the present embodiment, as a wire-driven mechanism has the characteristics of small frictional force, zero return stroke, uniform stress, and the like, the joint friction can be effectively reduced, the deceleration return stroke is eliminated, the reverse driving performance of the hand structure 3 is improved, the return stroke clearance is reduced, and the improvement of the end flexibility and operation feeling of the position elbow pitching joint 23 is facilitated.

As shown in Fig. 1 and Fig. 2, in the present embodiment, in order to make the hand structure 3 move more flexibly and the control on the hand structure 3 be convenient, the posture redundancy deflection joint 31, the posture pitching joint 32, the posture deflection joint 33, and the posture rotation joint 34 all adopt structures with relatively small overall sizes. Because of their relatively small installation space, the posture redundancy deflection joint 31, the posture pitching joint 32, the posture deflection joint 33 and the posture rotation joint 34 all adopt gear transmission mechanisms. Therefore, each joint can realize the self-balance of self-gravity through the feedback adjustment of the motors while improving the torque, and is ensured to be capable of stopping at any point in the range of motion without external force, and the free movement of each joint can be realized through a software algorithm.

As shown in Fig. 1 and Fig. 2, in the present embodiment, the clip pinching joint 35 can realize the clamping action of a clip and independently corresponds to the pinching action of the clip in a surgical instrument by adopting the multi-link design, and the main manipulator adopts the bionic design as a whole, and has good similarity with human arms and wrists, and good flexibility.

It should be noted that in order to ensure that the main manipulator has a good reverse driving performance, the frictional force at position joints of the main manipulator should be relatively small, but at the same time, in order to meet the load requirements, the position joints of the main manipulator need to have a certain reduction ratio. Therefore, the deceleration mechanisms of the position shoulder pitching joint 12 and the position elbow pitching joint 23 (the two joints with the largest load) can adopt secondary wire-driven structures. As for posture joints, because of low load requirements and limited joint space, it is not suitable to adopt a wire-driven mode, so a gear transmission mode is adopted, such as planetary gear deceleration structures or bevel gear transmission structures.

It should be noted that the main manipulator is a typical human-computer interaction robotic arm, and the joints can usually be driven by DC (direct-current) servo motors. This method is mature, simple in energy, wide in speed variation range, high in efficiency and very high in speed and position accuracy.

It should be noted that in order to realize the full closed-loop control of the main manipulator and avoid zero point seeking, it is necessary to install encoders, such as absolute value encoders, on the position joints of the main manipulator. At the same time, in order to realize double backup code wheels to improve the position accuracy and the braking safety, an incremental encoder can also be installed at the rear end of each position joint driving motor.

Another embodiment of the invention provides surgical robot control equipment, which includes a console 100 and the above-mentioned main manipulator. After the root structure 1 of the main manipulator is connected to the chassis 101 of the console 100, the hand structure 3 of the main manipulator is suitable for being exposed from the table top of the console 100.

Optionally, the surgical robot control equipment further includes a display module 200 and an armrest module 300. The display module 200 is arranged on the console 100 and located on one side of the main manipulator. The armrest module 300 is arranged on the console 100 and located on one side, away from the display module 200, of the main manipulator.

As shown in Fig. 4, Fig. 5 and Fig. 6, in the present embodiment, the display module 200 includes a main display 201 and an auxiliary display 202; the console 100 includes the chassis 101 and a stand column 102; the stand column 102 is arranged at the middle of the front of the chassis 101; the main display 201 can be directly arranged on the upper part of the stand column 102 through a support; a transverse supporting rod is also connected to the stand column 102; and the auxiliary display 202 is arranged at one side of the main display 201 and can be installed on the transverse supporting rod through another support.

It should be noted that the stand column 102 adopts the liftable stand column, which is convenient for adjustment of the height of the display module 200 to meet the usage needs of operators with different heights. The transverse supporting rod can adopt the structure with movable joints, which is convenient for adjustment of the position and angle of the auxiliary display 202.

The main manipulator is arranged at the rear side of the display module 200 in the height direction of the console 100, and the root structure 1 of the main manipulator is connected to the chassis 101, so that the main manipulator is positively arranged on the console 100. The hand structure 3 of the main manipulator can be exposed from the table top and is lower than the lower edge of the display module 200, therefore, the main manipulator does not shield the display module 200, so that the operator can conveniently observe the display module 200 when operating the surgical robot control equipment.

It should be noted that the positive arrangement refers to the arrangement mode in which the main manipulator with the up-down structure is arranged on the chassis 101 in the height direction of the console 100, which is different from another arrangement mode in which the main manipulator is hoisted on a cantilever under normal circumstances.

It is characterized in that, as shown in Fig. 4, the height direction of the console 100 refers to the Z-axis direction.

It should be noted that the main manipulator is not limited to be positively arranged on the console 100 in the present embodiment, but also can be positively arranged on other surgical equipment involving surgical control, such as doctor's consoles or remote devices.

As shown in Fig. 4, Fig. 5 and Fig. 6, in the present embodiment, the armrest module 300 includes an armrest 301, a touch screen 302 and keys 303. The armrest 301 is arranged at the rear of the chassis 101 of the console 100 and includes a platform, a left leg and a right leg. One end of the left leg is connected to the left end of the rear of the chassis 101, and the other end of the left leg is connected to the left end of the platform. One end of the right leg is connected to the right end of the rear of the chassis 101, and the other end of the right leg is connected to the right end of the platform. The touch screen 302 and the keys 303 are all arranged on the platform. A pedal 400 is arranged at a position, corresponding to the platform of the armrest 301, of the chassis; and during operation, arms can be placed on the armrest 301, and feet step on the pedal 400, so that the operator can control the manipulator with a more comfortable control posture, and thus, fatigue operation is avoided.

In addition, as shown in Fig. 4, Fig. 5 and Fig. 6, in the present embodiment, a push hand 500 is arranged under the display module 1 on the stand column 22, and is of an annular structure, which can avoid obstructing the operation of the main manipulator. A voice device 600 is arranged at a position, located between the left main manipulator and the right manipulator and directly facing the operator, of the push hand 500, and can facilitate the operator to make voice calls with an operating room and know the operation information in real time.

As shown in Fig. 4, Fig. 5 and Fig. 6, in the present embodiment, in order to prevent the armrest module 300 from interfering with the control of the main manipulator, meanwhile provide a better control posture for the operator, and adapt to operators in different shapes for operation, the horizontal distance between the edge of the hand structure 3 and the armrest 301 can be set between 200 mm and 240 mm, for example, 220 mm, and the vertical distance between the top of the hand structure 3 and the armrest 301 can be set between 80 mm and 120 mm, for example, 100 mm.

Compared with traditional control equipment with the main manipulator installed in the form of hoisting, the surgical robot control equipment in the present embodiment has lower requirements on the rigidity of the armrest module 300 and the whole machine. The positive arrangement mode of the main manipulator on the chassis can reduce the processing difficulty of the equipment and the resonance phenomenon resulting from the hoisting mode. At the same time, the positive arrangement mode on the chassis makes the center of gravity of the whole machine lower, the stability of the surgical robot control equipment better, the self-balancing design of the main manipulator simpler and more reliable, and the structure be optimized to be smaller, thus making the operation more flexible.

In addition, the positive arrangement mode of the main manipulator on the chassis can also ensure that the three-dimensional display has the best observation distance. The whole main manipulator adopts bionic design, has good similarity with human arms and wrists, good flexibility and is relatively smooth in operation.

Although the the invention is disclosed as above, the scope of protection of the invention is not limited thereto. Those skilled in the art can make various changes and modifications without departing from the spirit and scope of the the invention, and all these changes and modifications shall fall within the scope claimed by the invention.

## Claims

1. A main manipulator, **characterized by** comprising a root structure (1), an arm structure (2), and a hand structure (3), wherein the root structure (1) is suitable for being connected to a chassis (101) of a console (100); one end of the arm structure (2) is connected to the root structure (1), and the other end of the arm structure (2) is connected to the hand structure (3); and after the root structure (1) is connected to the chassis (101), the hand structure (3) is suitable for being exposed from the table top of the console (100).

2. The main manipulator according to claim 1, **characterized in that** the root structure (1) comprises a position shoulder deflection joint (11) which is suitable for being connected to the chassis (101) and realizing a position shoulder deflection operation.

3. The main manipulator according to claim 2, **characterized in that** the root structure (1) further comprises a position shoulder pitching joint (12) which is suitable for being connected to the position shoulder deflection joint (11) and realizing a position shoulder pitching operation.

4. The main manipulator according to claim 3, **characterized in that** the arm structure (2) comprises a big arm (21), a small arm (22) and a position elbow pitching joint (23), wherein one end of the big arm (21) is connected to the position shoulder pitching joint (12), and the other end of the big arm (21) is connected to the small arm (22) through the position elbow pitching joint (23); and the position elbow pitching joint (23) is suitable for realizing a position elbow pitching operation.

5. The main manipulator according to claim 4, **characterized in that** the hand structure (3) comprises a posture redundancy deflection joint (31), a posture pitching joint (32), a posture deflection joint (33) and a posture rotation joint (34) which are connected in sequence through a skeleton structure, wherein the posture redundancy deflection joint (31) is connected to one end, far away from the position elbow pitching joint (23), of the small arm (22), and is suitable for realizing a posture redundancy deflection operation; the posture pitching joint (32) is suitable for realizing a posture pitching operation; the posture deflection joint (33) is suitable for realizing a posture deflection operation; and the posture rotation joint (34) is suitable for realizing a posture rotation operation.

6. The main manipulator according to claim 5, **characterized in that** the joint axes of the posture redundancy deflection joint (31), the posture pitching joint (32), the posture deflection joint (33), and the posture rotation joint (34) intersect at one point.

7. The main manipulator according to claim 5 or 6, **characterized in that** the hand structure (3) further comprises a clip pinching joint (35) which is connected to the posture rotation joint (34) and is suitable for realizing a clip pinching operation.

8. Surgical robot control equipment, **characterized by** comprising the console (100) and the main manipulator according to any one of claims 1 to 7, wherein after the root structure (1) of the main manipulator is connected to the chassis (101) of the console (100), the hand structure (3) of the main manipulator is suitable for being exposed from the table top of the console (100).

9. The surgical robot control equipment according to claim 8, **characterized by** comprising a display module (200) which is arranged on the console (100) and located on one side of the main manipulator.

10. The surgical robot control equipment according to claim 9, **characterized by** further comprising an armrest module (300) which is arranged on the console (100) and located on one side, away from the display module (200), of the main manipulator.
